# EUROPÄISCHE PATENTSCHRIFT

(11) **EP 3 635 348 B1**
(45) Veröffentlichungstag und Bekanntmachung des Hinweises auf die Patenterteilung: **15.12.2021**
(21) Anmeldenummer: 18720545.5
(22) Anmeldetag: 18.04.2018
(51) Int. Cl.: G01J 1/42, A61N 5/06

(54) **VERFAHREN UND VORRICHTUNG ZUR KALIBRATION EINER LICHTQUELLE EINER MEDIZINISCHEN VORRICHTUNG**
METHOD AND DEVICE FOR CALIBRATING A LIGHT SOURCE OF A MEDICAL DEVICE
PROCÉDÉ ET DISPOSITIF SERVANT À ÉTALONNER UNE SOURCE DE LUMIÈRE D'UN DISPOSITIF MÉDICAL

(30) Priorität: 07.06.2017 DE 102017112483
(43) Veröffentlichungstag der Anmeldung: 15.04.2020
(73) Patentinhaber: Omicron-Laserage Laserprodukte GmbH, 63110 Rodgau-Dudenhofen (DE)
(72) Erfinder: DINGES, Dieter, Oberursel (DE); FÜRSTENBERG, Wolfgang, 63867 Johannesberg (DE); BAUMANN, Sönke-Nils, 63739 Aschaffenburg (DE)
(74) Vertreter: Patentanwälte Olbricht Buchhold Keulertz
(86) Internationale Anmeldenummer: PCT/EP2018/059878
(87) Internationale Veröffentlichungsnummer: WO 2018/224211

(56) Entgegenhaltungen:
- US-A- 5 115 126
- US-A1- 2003 107 726
- GRANJON Y ET AL: "AUTOMATIC MEASUREMENT OF EMITTING PROFILE OF DIFFUSING TIP ON A MEDICAL LASER INSTRUMENT", MEDICAL AND BIOLOGICAL ENGINEERING AND COMPUTING, SPRINGER, HEILDELBERG, DE, Bd. 32, Nr. 3, 1. Mai 1994 (1994-05-01), Seiten 323-327, XP000451959, ISSN: 0140-0118

## Beschreibung

Die Erfindung betrifft ein Verfahren zur Kalibration einer Lichtquelle einer medizinischen Vorrichtung gemäß Anspruch 1, sowie eine entsprechende medizinische Vorrichtung gemäß Anspruch 8.

Im Stand der Technik sind heutzutage vielfältige Anwendungen von Lasersystemen im medizinischen Bereich bekannt. Neben den gängigen Anwendungen, wie beispielsweise in der Chirurgie oder in der Augenheilkunde, wird die durch Lasersysteme erzeugte Strahlung zunehmend auch in der Krebstherapie, beispielsweise im Rahmen der photodynamischen Therapie (PDT) verwendet. Dabei wird ein photosensitives Medikament verwendet, welches durch Aktivierung mit Licht einer bestimmten Wellenlänge aufgrund von photophysikalischen Prozessen Wirkstoffe freisetzt, welche beispielsweise Tumorzellen oder Bakterien angreifen. Dabei ist zur Aktivierung entsprechender Medikamente Licht einer bestimmten Wellenlänge und einer bestimmten Intensität (Irradiance) notwendig. Hierzu kann sowohl Laserlicht, als auch andere elektromagnetische Strahlung verwendet werden, welche eine ausreichend schmale spektrale Bandbreite aufweist.

Bei den Behandlungsmöglichkeiten der photodynamischen Therapie wird generell zwischen der oberflächlichen Behandlung und der invasiven (insbesondere der interstitiellen) Behandlung unterschieden. In beiden Bereichen wird jedoch zumeist die für die Behandlung verwendete elektromagnetische Strahlung in eine lichtleitende Faser eingekoppelt und am Ort der Behandlung aus der Faser ausgekoppelt. Je nach Behandlungstyp unterscheiden sich dabei die verwendeten Fasern in ihren Abstrahleigenschaften.

Um zu gewährleisten, dass bei einer Behandlung das behandelte Gewebe tatsächlich mit Licht der notwendigen Intensität beaufschlagt wird, ist es im Stand der Technik bekannt, eine Lichtquelle vor Behandlung zu kalibrieren. Hierbei wird im Allgemeinen bei der Kalibration verglichen, ob die aus einer Faser ausgekoppelte gesamte Lichtleistung der in die Faser eingekoppelten Leistung so weit entspricht, dass am Ort der Behandlung die gewünschte Lichtleistung zur Verfügung steht. Ist dies nicht der Fall kann die in die lichtleitende Faser eingekoppelte Lichtleistung so angepasst werden, dass die ausgekoppelte Lichtleistung den medizinischen Anforderungen entspricht. Es wird jedoch im Allgemeinen nicht unterschieden, aus welcher Art von Faser bzw. in welcher Art und Weise Licht aus der Faser ausgekoppelt wird. So kann es je nach Auslegung der Faser bei gleicher ausgekoppelter Lichtleistung zu unterschiedlichen aus der Faser ausgekoppelten Intensitäten kommen. Folglich besteht im Stand der Technik die Gefahr, dass zwar die insgesamt aus einer Faser ausgekoppelte Leistung nach einer Kalibration korrekt ist, jedoch aufgrund einer falsch gewählten Faser die Intensität zu hoch oder zu niedrig ist, sodass eine Behandlung nicht möglich, oder eine Verletzung des Patienten zu befürchten ist.

US 2003/0107726 A1 beschreibt ein Gerät zur Ermittlung von Transmissionsverlusten von insbesondere lichtleitenden Fasern eines Endoskopes. Dabei wird ein in einer Kammer angeordneter Lichtsensor zur Ermittlung der Lichtintensität eingesetzt, welche mit einer Referenzwert abgeglichen wird.

Auch US 5 115 126 A offenbart eine Vorrichtung zur Überprüfung einer Lichtquelle eines Endoskopes, welche Licht durch eine lichtleitende Verbindung zum beobachteten Objekt leitet. Mittel zur Ermittlung der emittierten Lichtmenge ermöglichen eine Soll-/Ist-Wertabgleich zur Bestimmung einer korrekten Funktionsfähigkeit der Vorrichtung.

Granjon Y et al.: "Automatic measurement of emitting profile of diffusing tip on a medical laser instrument", Medical and Biological Engineering and Computing, Springer, Heidelberg, Germany, Band 32, Nr. 3, 1. Mai 1994 beschreibt eine Vorrichtung zur Vermessung der Lichtstreuung einer Faserspitze, wobei die Messelemente in Form von Photodioden auf einem automatisierten mobilen Element angeordnet sind, welches um die emittierende Faserspitze rotierbar ist. Demgegenüber liegt der vorliegenden Erfindung die Aufgabe zugrunde ein Verfahren zur Kalibration einer Lichtquelle einer medizinischen Vorrichtung zu schaffen, bei der eine Fehlbehandlung aufgrund einer falsch gewählten ausgekoppelten Intensität der verwendeten elektromagnetischen Strahlung vermieden wird.

Hauptmerkmale der Erfindung sind in Anspruch 1 sowie in dem nebengeordneten Anspruch angegeben. Ausgestaltungen sind Gegenstand der Ansprüche 2 bis 7.

In einem ersten Aspekt betrifft die Erfindung ein Verfahren zur Kalibration einer Lichtquelle einer medizinischen Vorrichtung, wobei die Lichtquelle mit wenigstens einer lichtleitenden Faser so verbindbar ist, dass von der Lichtquelle erzeugte elektromagnetische Strahlung einer definierten Lichtleistung in die lichtleitende Faser zumindest teilweise eingekoppelt wird. Die medizinische Vorrichtung ist mit wenigstens einem Kalibrationsport verbunden, wobei der Kalibrationsport Sensormittel zur Ermittlung einer räumlichen Abstrahlcharakteristik einer in den Kalibrationsport eingeführten lichtleitenden Faser aufweist. Das Verfahren weist dabei die nachfolgenden Schritte auf.

Zunächst wird die lichtleitende Faser mit der Lichtquelle verbunden und eine Positioniervorrichtung für die lichtleitende Faser in den Kalibrationsport eingesetzt, wobei die Positioniervorrichtung einen Aufnahmekanal zur Aufnahme der lichtleitenden Faser sowie wenigstens eine Abstrahlöffnung aufweist, wobei die wenigstens eine Abstrahlöffnung die Transmission einer aus der lichtleitenden Faser ausgekoppelten Lichtleistung in wenigstens einer definierten Raumrichtung ermöglicht. Anschließend wird die lichtleitende Faser in den Aufnahmekanal der Positioniervorrichtung eingeführt und elektromagnetische Strahlung einer definierten Lichtleistung in die lichtleitende Faser eingekoppelt. Dann wird die räumliche Abstrahlcharakteristik der aus der lichtleitenden Faser im Bereich des Kalibrationsports ausgekoppelten Lichtleistung mittels der Sensormittel als Ist-Abstrahlcharakteristik sowie eine Soll-Abstrahlcharakteristik der aus der lichtleitenden Faser im Bereich des Kalibrationsports ausgekoppelten Lichtleistung für die in die lichtleitende Faser eingekoppelte Lichtleistung ermittelt. Die ermittelte Ist-Abstrahlcharakteristik wird dann mit der Soll-Abstrahlcharakteristik verglichen, und wenn die Ist-Abstrahlcharakteristik der Soll-Abstrahlcharakteristik entspricht, die lichtleitende Faser zur weiteren Verwendung freigegeben. Wenn hingegen die Ist-Abstrahlcharakteristik der Soll-Abstrahlcharakteristik nicht entspricht, endet das Verfahren mit dem Ausgeben einer Fehlermeldung.

Bei einer "Lichtquelle" kann es sich dabei um eine beliebige Quelle für elektromagnetische Strahlung handeln, welche eine ausreichend schmale spektrale Bandbreite aufweist, sodass sie beispielsweise für die Verwendung in der PDT geeignet ist. Dabei kann es sich beispielsweise um LEDs, insbesondere um Laserdioden zur Erzeugung von Laserstrahlung handeln. Die von der Lichtquelle erzeugte Strahlung wird dabei vorzugsweise mittels einer entsprechenden Optik in eine lichtleitende Faser, beispielsweise eine Glasfaser, eingekoppelt, welche beispielsweise einen Durchmesser von 400 µm bis 600 µm aufweisen kann. Vorzugsweise wird im Rahmen des beschriebenen Verfahrens Laserlicht als elektromagnetische Strahlung verwendet.

Eine Abstrahlcharakteristik beschreibt in diesem Kontext die Verhältnisse von Lichtleistungen, welche aus der lichtleitenden Faser in verschiedenen Raumrichtungen abgestrahlt werden unter Berücksichtigung einer in die lichtleitende Faser eingekoppelten Lichtleistung. Das Übereinstimmen der Ist-Abstrahlcharakteristik mit der Soll-Abstrahlcharakteristik kann dabei unter Berücksichtigung einer definierten Toleranz ermittelt werden. Beispielsweise kann von einem "Übereinstimmen" der Charakteristika gesprochen werden, wenn die jeweiligen Verhältnisse um weniger als 5% voneinander abweichen. Das Toleranzniveau kann dabei für verschiedene Verhältnisse unterschiedlich gewählt sein.

Das vorstehend beschriebene Verfahren hat dabei den Vorteil, dass nicht die absolute aus einer lichtleitenden Faser ausgekoppelte Lichtleistung zur Kalibration der Lichtquelle herangezogen wird, sondern vielmehr die Abstrahlcharakteristik der aus der lichtleitenden Faser ausgekoppelten Strahlung. Aus der Abstrahlcharakteristik der ermittelten Strahlung lässt sich nämlich ableiten, welche Art von lichtleitender Faser aktuell mit der Lichtquelle verbunden ist. Ist dabei für eine bestimmte Behandlungsmethode oder Faserart die Soll-Abstrahlcharakteristik bekannt, kann durch einen Vergleich der Charakteristika erkannt werden, ob die richtige Faser mit der Lichtquelle verbunden ist.

Stimmt dabei die Soll-Abstrahlcharakteristik mit der Ist-Abstrahlcharakteristik nicht überein, kann dies mehrere Ursachen haben. Entweder ist die falsche Faser bzw. der falsche Fasertyp mit der Lichtquelle verbunden, oder die Positioniervorrichtung, welche in dem Kalibrationsport eingesetzt ist, passt nicht mit der verwendeten lichtleitenden Faser zusammen, da sich die durch die Positioniervorrichtung freigegebenen Raumrichtungen nicht mit der Abstrahlcharakteristik der lichtleitenden Faser deckt. Nur bei Verwendung der korrekten Faser in Kombination mit der korrekten Positioniervorrichtung wird die Ist-Abstrahlcharakteristik mit der Soll-Abstrahlcharakteristik übereinstimmen, sodass die Faser für eine weitere Verwendung freigegeben werden kann.

Bei einer "weiteren Verwendung" kann es sich beispielsweise um die Anpassung der in die lichtleitende Faser eingekoppelten Lichtleistung handeln, sodass eine Behandlung aufgrund einer ausreichenden aus der lichtleitenden Faser ausgekoppelten Intensität gestartet werden kann. Sofern die aus der lichtleitenden Faser ausgekoppelte Lichtleistung bereits für eine Behandlung ausreichend ist, kann als weitere Verwendung auch die Behandlung eines Patienten direkt gestartet werden.

Die im Falle einer nicht übereinstimmenden Ist-Abstrahlcharakteristik mit der Soll-Abstrahlcharakteristik ausgegebene Fehlermeldung kann dabei eine Information enthalten, welche Fehlerquelle vermutlich vorliegt. So kann anhand der Abweichungen beispielsweise ermittelt werden, ob die falsche Faser oder die falsche Positioniervorrichtung verwendet wurde. Die für den Vergleich der Charakteristika verwendete Soll-Abstrahlcharakteristik kann dabei beispielsweise aus einem Speicher der medizinischen Vorrichtung ausgelesen werden und auf Grundlage einer Information bezüglich der verwendeten lichtleitenden Faser definiert sein.

Bei Ausgabe einer Fehlermeldung kann nach einer Ausführungsform vorgesehen sein, dass die medizinische Vorrichtung automatisch in einen sicheren Zustand versetzt wird, in dem eine weitere Abstrahlung von elektromagnetische Strahlung durch die Lichtquellen verhindert wird. Dabei kann vorgesehen sein, dass dieser sichere Zustand erst dann wieder aufgehoben wird, wenn eine Änderung der gegenwärtigen Konfiguration der medizinischen Vorrichtung erfolgt. Eine solche Änderung kann beispielsweise ein Austausch der lichtleitenden Faser oder der Positioniervorrichtung, oder eine Änderung der in die lichtleitende Faser eingekoppelten Lichtleistung sein. Die Fehlermeldung kann dabei einen Hinweis enthalten, dass das Gerät erst nach einer solchen Änderung der Konfiguration wieder in Betrieb genommen werden kann.

Die einzelnen Verfahrensschritte des erfindungsgemäßen Verfahrens können dabei, soweit logisch sinnvoll, auch in einer anderen Reihenfolge ausgeführt werden. So kann beispielsweise das Einsetzen der Positioniervorrichtung in den Kalibrationsport durchaus auch vor dem Verbinden der lichtleitenden Faser mit der Lichtquelle erfolgen. Gleichermaßen kann auch eine Soll-Abstrahlcharakteristik ermittelt werden, bevor die Ist-Abstrahlcharakteristik bekannt ist.

Wie zuvor bereits ausgeführt wurde, können sich die verwendeten lichtleitenden Fasern in ihrer Abstrahlcharakteristik unterscheiden. Dabei ist nach einer Ausführungsform vorgesehen, dass es sich bei einer lichtleitenden Faser um eine Faser eines ersten Fasertyps oder eines zweiten Fasertyps handelt, wobei der erste Fasertyp dazu ausgebildet ist, in die lichtleitende Faser eingekoppelte elektromagnetischen Strahlung in Längsrichtung der Faser zu emittieren und wobei der zweite Fasertyp dazu ausgebildet ist, in die lichtleitende Faser eingekoppelte elektromagnetische Strahlung quer zur Längsrichtung der Faser über eine definierte Länge der Faser zu emittieren. In diesem Fall weist das Verfahren ferner das Ermitteln des verwendeten Fasertyps auf, wobei die Ermittlung der Soll-Abstrahlcharakteristik den ermittelten Fasertyp berücksichtigt.

Bei dem zweiten Fasertyp kann dabei vorgesehen sein, dass bei dem Ermitteln des Fasertyps gleichzeitig auch die Länge der Faser ermittelt wird, über die elektromagnetische Strahlung in radialer Richtung aus der Faser ausgekoppelt wird. Ferner können in einer Information bezüglich eines Fasertyps auch weitere Faserparameter kodiert sein. Beispielsweise kann bei der Ermittlung eines ersten Fasertyps gleichzeitig eine Information bezüglich der Form des Faserquerschnitts erhalten werden.

Zur Ermittlung des Fasertyps kann beispielsweise vorgesehen sein, dass die Faser mit einem RFID-Chip oder einem ähnlichen Identifikationsmerkmal ausgestattet ist, aus dem der Fasertyp abgeleitet werden kann. In diesem Fall kann beispielsweise die medizinische Vorrichtung in dem Moment, in dem die Faser mit der Lichtquelle verbunden wird, durch auslesen des RFID-Chips selbstständig ermitteln, welcher Fasertyp angeschlossen wurde und dementsprechend die zu erwartende Soll-Abstrahlcharakteristik auswählen. Dies hat den Vorteil, dass eine Fehlerquelle, nämliche eine fehlerhafte Bedienung des Gerätes im Sinne einer fehlerhaften Auswahl der angeschlossenen Faser, vermieden werden kann.

Alternativ zu einer automatischen Erkennung des Fasertyps ist nach einer weiteren Ausführungsform vorgesehen, dass das Ermitteln des verwendeten Fasertyps eine Benutzereingabe umfasst. Um die Kalibration einer Lichtquelle einer medizinischen Vorrichtung weiter zu vereinfachen, ist nach einer weiteren Ausführungsform vorgesehen, dass das Verfahren ferner das Ermitteln der in die lichtleitende Faser einzukoppelnden definierten Lichtleistung auf Grundlage des an die Lichtquelle angeschlossenen Fasertyps beinhaltet. In diesem Fall kann beispielsweise vorgesehen sein, dass durch einen Benutzer oder die medizinische Vorrichtung selbst eine aus der lichtleitenden Faser auszukoppelnde Intensität vorgegeben wird, welche automatisch durch die medizinische Vorrichtung anhand einer Information über die zu verwendende lichtleitende Faser in eine in die Faser einzukoppelnde Lichtleistung umgerechnet wird. Hierdurch wird die Wahrscheinlichkeit einer Fehlbedienung weiter verringert.

Nach einer weiteren Ausführungsform ist es auch möglich, dass durch einen Nutzer lediglich eine bestimmte Therapieart sowie die Größe einer zu behandelnden Fläche vorgegeben wird, woraufhin die medizinische Vorrichtung selbstständig ermittelt, welcher Fasertyp verwendet werden muss und welche Lichtleistung in die Faser eingekoppelt werden soll, um die für die Behandlung notwendige Intensität aus der Faser auskoppeln zu können. Dementsprechend wird durch die medizinische Vorrichtung aus der ausgewählten Therapieart auch ermittelt, welche Soll-Abstrahlcharakteristik zu erwarten ist. Folglich kann die Definition der Therapieparameter vollständig automatisiert werden, sodass eine Fehlbedienung nahezu ausgeschlossen wird.

Erfindungsgemäss weisen die Sensormittel wenigstens eine seitliche Photodiode auf, die seitlich an dem Kalibrationsport angeordnet ist sowie wenigstens eine frontale Photodiode, die an einem longitudinalen Ende des Kalibrationsports angeordnet ist. Erfindungsgemäss umfasst das Ermitteln der Ist-Abstrahlcharakteristik das Ermitteln des in den Photodioden durch die von der lichtleitenden Faser ausgekoppelte Lichtleistung erzeugten jeweiligen Photostroms, wobei das Vergleichen der ermittelten Ist-Abstrahlcharakteristik mit der ermittelten Soll-Abstrahlcharakteristik das Vergleichen der ermittelten Photoströme mit in der Soll-Abstrahlcharakteristik enthaltenen Photoströmen für die jeweiligen Photodioden umfasst.

Die Verwendung wenigstens einer seitlichen Photodiode sowie einer frontalen Photodiode erlaubt anhand der ermittelten Photoströme einen dezidierten Vergleich der Abstrahlung longitudinal und transversal zu einer verwendeten lichtleitenden Faser. Vorzugsweise sind dabei zwei seitliche Photodioden vorgesehen, welche sich in den Kalibrationsport diametral gegenüberliegen. Durch eine solche Anordnung ist eine bessere Auflösung der Abstrahlcharakteristik der lichtleitenden Faser möglich. Dabei ist es sowohl möglich, die Photodioden bzw. die in den Photodioden erzeugten Photoströme zur Bestimmung einer Abstrahlcharakteristik kombiniert, als auch getrennt zu betrachten. Je nach Anzahl der verwendeten Photodioden können auch beliebige Kombinationen von Photoströmen unterschiedlich platzierter Photodioden in die Ermittlung der Abstrahlcharakteristik einfließen.

Bei Verwendung der Photoströme zur Ermittlung der Soll- und Ist-Abstrahlcharakteristik ist nach einer weiteren Ausführungsform vorgesehen, dass, wenn die Verhältnisse der Photoströme der Ist-Abstrahlcharakteristik den Verhältnissen der Photoströme der Soll-Abstrahlcharakteristik entsprechen, die Photoströme der Ist-Abstrahlcharakteristik jedoch geringer sind als die entsprechenden Photoströme der Soll-Abstrahlcharakteristik, die Fehlermeldung einen Defekt der lichtleitenden Faser und/oder der Kopplung zwischen der lichtleitenden Faser und der Lichtquelle anzeigt. Stimmt nämlich die Ist-Abstrahlcharakteristik an sich, welche wie zuvor beschrieben, die Verhältnisse von ermittelten Lichtleistungen beschreibt, mit der Soll-Abstrahlcharakteristik überein, ist die verwendete lichtleitende Faser in Kombination mit der verwendeten Positioniervorrichtung korrekt gewählt. Sind jedoch die Absolutwerte der Photoströme insgesamt zu niedrig, deutet dies auf einen erhöhten Verlust von Lichtleistung in der Faser oder im Bereich zwischen Lichtquelle und lichtleitender Faser hin. Dies ist insbesondere dann der Fall, wenn die medizinische Vorrichtung dazu ausgebildet ist, selbstständig die in die lichtleitende Faser einzukoppelnde Lichtleistung aufgrund einer Wahl einer gewünschten Therapievariante durch einen Benutzer zu wählen.

Zuvor wurde beschrieben, dass die medizinische Vorrichtung eine Lichtquelle aufweist, welche mit wenigstens einer lichtleitenden Faser verbunden und anschließend entsprechend dem erfindungsgemäßen Verfahren kalibriert werden kann. Nach einer weiteren Ausführungsform ist dabei vorgesehen, dass die medizinische Vorrichtung wenigstens zwei Lichtquellen aufweist, die jeweils mit einer lichtleitenden Faser so verbindbar sind, dass von den Lichtquellen erzeugte elektromagnetische Strahlung jeweils in die mit einer Lichtquelle verbundene lichtleitende Faser zumindest teilweise eingekoppelt wird. In diesem Fall werden die Verfahrensschritte individuell für die mit den Lichtquellen verbundenen lichtleitenden Fasern durchgeführt, wobei das Einsetzen einer Positioniervorrichtung nicht zwingend für jede lichtleitende Faser durchgeführt werden muss. So ist es durchaus möglich, dass an unterschiedlichen Lichtquellen identische Fasern verwendet werden, sodass ein Austausch der Positioniervorrichtung zwischen den Kalibrationsvorgängen nicht notwendig ist. Sollten sich hingegen die mit den individuellen Lichtquellen verbundenen lichtleitenden Fasern in ihrer Abstrahlcharakteristik aufgrund unterschiedlicher Abstrahlrichtungen (frontal oder radial) unterscheiden, muss auch die entsprechende Positioniervorrichtung, welche in dem Kalibrationsport eingesetzt ist, bei Durchführung des Kalibrationsvorgangs getauscht werden. Auf diese Weise kann eine Vielzahl von lichtleitenden Fasern parallel kalibriert werden, sodass eine Behandlung in mehreren zu behandelnden Bereichen eines Patienten parallel möglich wird.

Um die Bedienung der medizinischen Vorrichtung bzw. die Durchführung des Kalibrationsverfahrens weiter zu vereinfachen, ist nach einer weiteren Ausführungsform vorgesehen, dass nach der Freigabe einer Faser die mit der Faser verbundene Lichtquelle für eine definierte Zeitspanne Licht einer Wellenquelle zwischen 350 nm und 850 nm in die Faser einkoppelt. Effektiv leuchtet dann eine bereits kalibrierte lichtleitende Faser für eine Zeitspanne, beispielsweise 5 Minuten, im für den Menschen sichtbaren elektromagnetischen Spektrum weiter, sodass ein Anwender leicht erkennen kann, welche Fasern bereits kalibriert sind und welche noch nicht. Ein weiterer Vorteil dieser Ausführungsform besteht darin, dass nach Durchführung einer Kalibration die Sensormittel des Kalibrationsports weiterhin Strahlung detektieren, sofern die kalibrierte Faser nicht aus dem Kalibrationsport entfernt wurde. In diesem Fall kann einem Benutzer eine Fehlermeldung angezeigt werden, dass die falsche Faser in den Kalibrationsport angeordnet ist oder die Kalibration der Fasern bereits abgeschlossen wurde. Auf diese Weise kann demnach eine Verwechselung der Fasern zwischen bereits kalibrierten und noch nicht kalibrierten Fasern vermieden werden.

Um die Anwendersicherheit des Verfahrens zu erhöhen ist nach einer weiteren Ausführungsform vorgesehen, dass bei Erkennung einer kurzfristigen Änderung der gemessenen Abstrahlcharakteristik während des Kalibrationsprozesses die medizinische Vorrichtung in einen sicheren Zustand schaltet, in dem beispielsweise die wenigstens eine Lichtquelle der medizinischen Vorrichtung abgeschaltet wird und zunächst nicht mehr durch einen Benutzer aktivierbar ist. Eine solche kurzfristige Änderung der gemessenen Abstrahlcharakteristik tritt beispielsweise auf, wenn die lichtleitende Faser während aus dem Kalibrationsport herausgezogen wird, oder an einer Position entlang der Faser durchtrennt wird. Durch das Abschalten der Lichtquellen kann vermieden werden, dass die aus der lichtleitenden Faser austretende elektromagnetische Strahlung zu Verletzungen bei einem Anwender führt. Der sichere Zustand der medizinischen Vorrichtung kann beispielsweise durch eine entsprechende Nutzereingabe wieder aufgehoben werden.

In einem weiteren Aspekt betrifft die Erfindung eine medizinische Vorrichtung mit wenigstens einer Lichtquelle, wobei die Lichtquelle mit wenigstens einer lichtleitenden Faser so verbindbar ist, dass von der Lichtquelle erzeugte elektromagnetische Strahlung einer definierten Lichtleistung in die lichtleitende Faser zumindest teilweise eingekoppelt wird. Die medizinische Vorrichtung ist mit wenigstens einem Kalibrationsport verbunden, wobei der Kalibrationsport Sensormittel zur Ermittlung einer räumlichen Abstrahlcharakteristik einer in den Kalibrationsport eingeführten lichtleitenden Faser aufweist und wobei die Vorrichtung dazu ausgebildet ist, elektromagnetische Strahlung einer definierten Lichtleistung in eine mit der Vorrichtung verbundene und in dem Kalibrationsport positionierte lichtleitende Faser einzukoppeln, die räumliche Abstrahlcharakteristik der aus der lichtleitenden Faser im Bereich des Kalibrationsports ausgekoppelten Lichtleistung als Ist-Abstrahlcharakteristik zu ermitteln, eine Soll-Abstrahlcharakteristik der aus der lichtleitenden Faser im Bereich des Kalibrationsports ausgekoppelten Lichtleistung für die in die lichtleitende Faser eingekoppelte Lichtleistung zu ermitteln, die ermittelte Ist-Abstrahlcharakteristik mit der ermittelten Soll-Abstrahlcharakteristik zu vergleichen und wenn die Ist-Abstrahlcharakteristik mit der Soll-Abstrahlcharakteristik entspricht, die lichtleitende Faser zur weiteren Verwendung freizugeben. Wenn die Ist-Abstrahlcharakteristik der Soll-Abstrahlcharakteristik nicht entspricht, ist die Vorrichtung ferner dazu ausgebildet, eine Fehlermeldung auszugeben.

Erfindungsgemäss ist vorgesehen, dass die Sensormittel wenigstens eine seitliche Photodiode aufweisen, die seitlich an den Kalibrationsport angeordnet ist sowie wenigstens eine frontale Photodiode, die an dem longitudinalen Ende des Kalibrationsports angeordnet ist. Die Vorrichtung ist dann zum Ermitteln der Ist-Abstrahlcharakteristik dazu ausgebildet, den in den Photodioden durch die von der lichtleitenden Faser ausgekoppelte Lichtleistung erzeugten jeweiligen Photostrom zu ermitteln und ist ferner zum Vergleichen der ermittelten Ist-Abstrahlcharakteristik mit der ermittelten Soll-Abstrahlcharakteristik dazu ausgebildet, die ermittelten Photoströme mit in der Soll-Abstrahlcharakteristik enthaltenen Photoströmen für die jeweiligen Photodioden zu vergleichen.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Figur 1: eine schematische Darstellung eines Systems zur Durchführung des erfindungsgemäßen Verfahrens,
- Figur 2: schematische Darstellungen eines Kalibrationsports mit einer Positioniervorrichtung und Faser, und
- Figur 3: ein Flussdiagramm des erfindungsgemäßen Verfahrens.

Im Folgenden werden einander ähnliche oder identische Merkmale mit denselben Bezugszeichen gekennzeichnet.

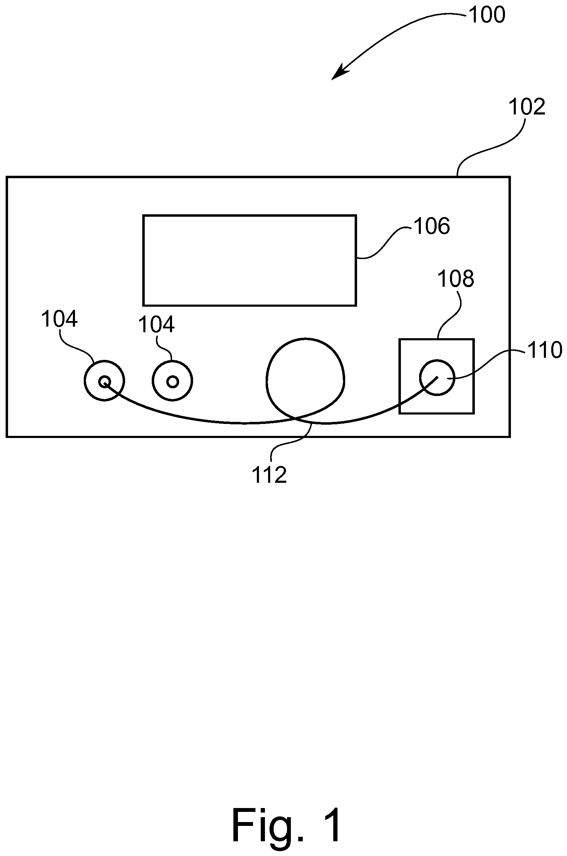

Die Figur 1 zeigt eine schematische Darstellung eines Systems 100, welches zur Durchführung des erfindungsgemäßen Verfahrens geeignet ist. Das System 100 weist hierzu eine medizinische Vorrichtung 102 auf, welche in der dargestellten Ausführung über zwei Lichtquellen 104, ein Bedienelement 106 und einen Kalibrationsport 108 verfügt. Bei dem Bedienelement 106 kann es sich beispielsweise um ein berührungssensitives Display handeln, welches sowohl zur Anzeige von Betriebsparametern, wie auch zur Einstellung von gewünschten Betriebsparametern durch einen Nutzer des Systems 100 geeignet ist. Obwohl in Figur 1 das Bedienelement 106 als einteiliges Element dargestellt ist, kann es auch vorgesehen sein, dass das Bedienelement 106 aus mehreren Elementen besteht. Beispielsweise kann zusätzlich zu dem Bedienelement 106 auch ein Not-Ausschalter an der medizinischen Vorrichtung 102 vorgesehen sein, mit der die Lichtquellen 104 kurzfristig abgeschaltet werden können.

Das System 100 umfasst weiter eine Positioniervorrichtung 110, welche in den Kalibrationsport 108 eingesetzt ist sowie in der dargestellten Ausführung eine lichtleitende Faser 112, welche einerseits mit einer Lichtquelle 104 verbunden ist und andererseits mit dem anderen Ende der lichtleitenden Faser 112 in die Positioniervorrichtung 110 eingesetzt ist. Dabei kann die lichtleitende Faser 112 beispielsweise über entsprechende Faserstecker mit den Lichtquellen 104 verbunden werden, beispielsweise über FC/PC- oder F-SMA-Stecker oder eine proprietäre Steckverbindung. Das System 100 kann beispielsweise im Rahmen einer photodynamischen Therapie eingesetzt werden, wie es bereits eingangs erwähnt wurde. Dabei wird einem Patienten zunächst ein Medikament verabreicht, welches eine photoaktivierbare Substanz beinhaltet. Wird diese Substanz mit Licht einer bestimmten Wellenlänge und Intensität bestrahlt, findet aufgrund photophysikalischer Prozesse eine Umwandlung bzw. Aktivierung eines Wirkstoffs der Substanz statt, sodass beispielsweise Bakterien oder Krebszellen von dem Wirkstoff angegriffen werden. Je nach Art der Behandlung werden dabei unterschiedliche Arten von lichtleitenden Fasern 112 und unterschiedliche Intensitäten in das zu behandelnde Gewebe eingebracht. Hierzu ist im Allgemeinen notwendig, dass die von den Lichtquellen 104 in die lichtleitende Faser 112 eingekoppelte Lichtleistung auf die Art der lichtleitenden Faser 112 sowie auf die zu behandelnde Fläche abgestimmt wird. Üblicherweise wird dabei bezüglich der Art der lichtleitenden Fasern 112 zwischen frontal abstrahlenden lichtleitenden Fasern 112 und radial abstrahlenden lichtleitenden Fasern 112 unterschieden, wobei bei den radial abstrahlenden Fasern die Abstrahlung über eine definierte Länge der Faser erfolgt.

Wird beispielsweise bei Verwendung einer radial abstrahlenden Faser 112 eine bestimmte Lichtleistung für eine bestimmte Länge des abstrahlenden Bereiches der Faser 112 gewählt, jedoch eine Faser 112 mit einer hiervon unterschiedlichen abstrahlenden Länge eingesetzt, ergibt sich eine von den eigentlichen gewünschten Parametern abweichende Intensität, welche aus der lichtleitenden Faser 112 bei einer Behandlung ausgekoppelt wird. Hierdurch kann entweder das Medikament nicht aktiviert werden oder es besteht die Gefahr von Verbrennungen in dem behandelten Gewebe aufgrund einer zu hohen Lichtleistung. Erfindungsgemäß soll eine solche Fehlbehandlung vermieden werden, indem die mit der Lichtquelle 104 verbundene lichtleitende Faser 112 vor der Freigabe für eine Behandlung zunächst mittels des Kalibrationsports 108 kalibriert wird.

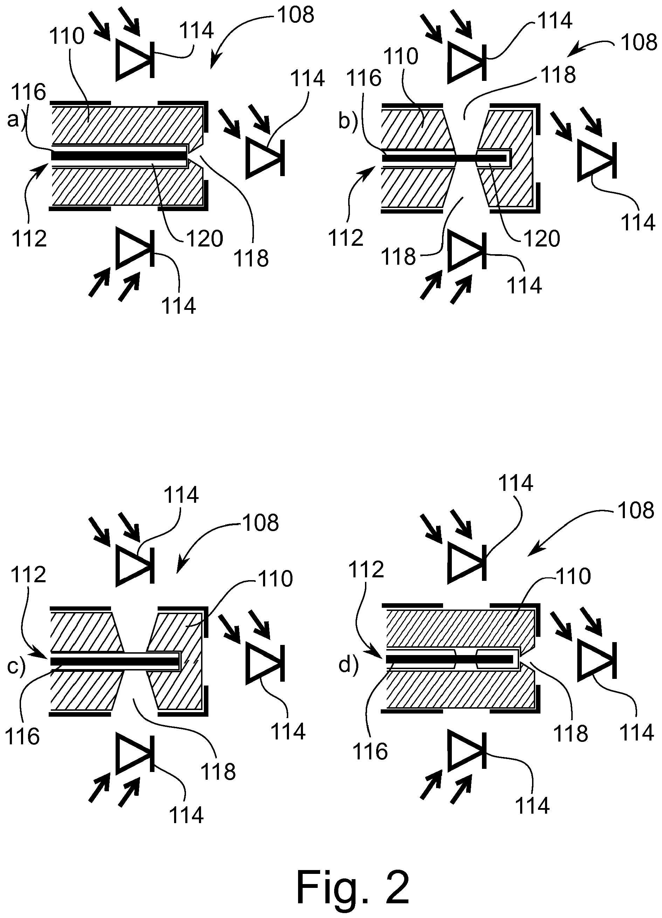

In Figur 2 sind verschiedene mögliche Konfigurationen von Kalibrationsports 108 mit darin eingesetzten Positioniervorrichtungen 110 und lichtleitenden Fasern 112 dargestellt, welche im Zuge einer Kalibration einer lichtleitenden Faser 112 auftreten können. In allen dargestellten Varianten weist der Kalibrationsport 108 drei Photodioden 114 auf, welche so in dem Kalibrationsport 108 angeordnet sind, dass sie innerhalb des Kalibrationsports 108 emittierte elektromagnetische Strahlung erfassen können und anhand eines hierdurch in den Photodioden erzeugten Photostroms quantifizieren können.

Bei dem Kalibrationsport 108 handelt es sich vorzugsweise um eine kreisrunde, z.B. zylindrisch geformte Ausnehmung, in die eine entsprechend geformte Positioniervorrichtung 110 eingesetzt werden kann. Die Positioniervorrichtung 110 ist beispielsweise ein aus Kunststoff gefertigter Körper, welcher für die aus der lichtleitenden Faser 112 austretende elektromagnetische Strahlung zumindest teilweise absorbierend wirkt. Beispielsweise kann die Positioniervorrichtung 110 aus Polyoxymethylen (POM) hergestellt sein. In den Figuren 2 a), 2 b), 2 c) und 2 d) sind verschiedene Kombinationen von Fasertypen und Positioniervorrichtungen 110 dargestellt.

In der Figur 2 a) ist eine lichtleitende Faser 112 dargestellt, welche an ihrem Faserende dazu ausgebildet ist, die in der lichtleitenden Faser 112 bzw. in dem Kern 120 der lichtleitenden Faser 116 geführte elektromagnetische Strahlung in frontaler Richtung der Faser 112 auszukoppeln. Die in Figur 2 a) in dem Kalibrationsport 108 angeordnete Positioniervorrichtung 110 ist dabei an diese Art der lichtleitenden Faser 112 angepasst. Hierzu weist die Positioniervorrichtung 110, welche vorzugsweise als rotationssymmetrischer Körper ausgeführt ist, an ihrem stirnseitigen Ende eine Ausnehmung 118 auf, durch die aus der lichtleitenden Faser 112 bzw. dem Faserkern 116 ausgekoppelte elektromagnetische Strahlung aus der Positioniervorrichtung 110 austreten kann.

Zur Bestimmung der Abstrahlcharakteristik der Kombination von Positioniervorrichtung 110 und lichtleitender Faser 112 werden die in den Photodioden 114 aufgrund der aus dem Kalibrationsport 108 austretenden elektromagnetischen Strahlung erzeugten Photoströme gemessen. Aufgrund der Art der lichtleitenden Faser 112 und der Geometrie der Positioniervorrichtung 110 ist zu erwarten, dass die am longitudinalen Ende des Kalibrationsports 108 angeordnete Photodiode 114 einen vergleichsweise starken Photostrom detektieren wird, während die seitlich an dem Kalibrationsport 108 angeordneten Photodioden 114 nur einen vergleichsweise geringen Photostrom detektieren werden, da ein Großteil der aus der lichtleitenden Faser 112 austretenden Strahlung in radialer Richtung durch das Material der Positioniervorrichtung 110 absorbiert wird. Dies entspricht der zu erwartenden Abstrahlcharakteristik für die korrekte Kombination von lichtleitender Faser 112 und Positioniervorrichtung 110. Dabei ist vorgesehen, dass die Positioniervorrichtung 110 teilweise transparent für die aus der lichtleitenden Faser 112 ausgekoppelte elektromagnetische Strahlung ist, sodass auch die Photodioden 114, welche an den Seiten des Kalibrationsports 108 angeordnet sind, mit elektromagnetischer Strahlung einer geringen Lichtleistung aufgrund von Streuung innerhalb der Positioniervorrichtung 110 beaufschlagt werden.

Die Figur 2 b) zeigt die Situation, in der in einem zu Figur 2 a) identischen Kalibrationsport 108 eine Positioniervorrichtung 110 für eine seitlich bzw. radial abstrahlende lichtleitende Faser 112 in Kombination mit einer solchen radial abstrahlenden lichtleitenden Faser 112 eingesetzt ist. Bei der lichtleitenden Faser 112 ist dabei die Ummantelung 120 der lichtleitenden Faser, welche im allgemeinen eine Abstrahlung von elektromagnetischer Strahlung vollständig abschirmt, entlang der Länge der Faser 112 über einen definierten Bereich geöffnet, sodass elektromagnetische Strahlung in radialer Richtung aus der Faser 112 austreten kann. Um zu ermöglichen, dass Strahlung radial aus der lichtleitenden Faser 112 austreten kann, kann der Faserkern 116 mit gezielten Mikroschädigungen als Streuzentren versehen sein, welche dazu ausgebildet sind, einfallende elektromagnetische Strahlung quer zur Faser 112 zu streuen. Die Streuzentren sind dabei vorzugsweise gleichmäßig im gesamten Faserkern 116 verteilt. Ferner kann der Faserkern 116 abschnittsweise durch ein Diffusormaterial ersetzt sein, welches einfallende elektromagnetische Strahlung quer zur Faser 112 streut. Bei dem Diffusormaterial kann es sich beispielsweise um ein Silikon mit darin enthaltenen lichtstreuenden Partikeln (Flakes) handeln. Die Positioniervorrichtung 110 ist dabei genau so ausgeführt, dass sie seitliche Ausnehmungen 118 aufweist, die bei einer vollständig in die Positioniervorrichtung 110 eingeführten lichtleitenden Faser 112 genau im Bereich des freiliegenden Faserkerns 116 angeordnet sind, sodass radial aus dem Faserkern 116 austretende elektromagnetische Strahlung von den seitlichen Photodioden 114 des Kalibrationsports 108 detektiert werden kann.

Am longitudinalen Ende der lichtleitenden Faser 112 ist hingegen die Faser 112 so ausgebildet, dass in longitudinaler Richtung der Faser 112 keine elektromagnetische Strahlung aus der Faser ausgekoppelt wird. Dies ist durch einen geschlossenen Fasermantel 120 dargestellt. Vorzugsweise wird am Ende der Faser ein die elektromagnetische Strahlung blockierendes Element, wie beispielsweise in Spiegel angeordnet, der dazu führt, dass noch nicht radial aus der Faser 112 ausgekoppelte Lichtleistung wieder zurück in den radial abstrahlenden Bereich der Faser 112 reflektiert wird. Hierdurch durchläuft die verbleibende elektromagnetische Strahlung nochmals den radial abstrahlenden Bereich der lichtleitenden Faser 112, sodass eine höhere und über die Länge des radial abstrahlenden Bereichs der Faser 112 homogenere radial ausgekoppelte Lichtleistung erreicht werden kann.

Die von der Kombination von Positioniervorrichtung 110 und lichtleitender Faser 112 gemäß Figur 2 a) zu erwartende Abstrahlcharakteristik besteht darin, dass die seitlich in dem Kalibrationsport 108 angeordneten Photodioden 114 einen vergleichsweise hohen Photostrom detektieren werden, während die frontal in dem Kalibrationsport 108 angeordnete Photodiode 114 nur einen sehr geringen Photostrom detektieren wird.

Bei einer erfindungsgemäßen Kalibration würde bei den Kombinationen, wie sie in Figur 2 a) und 2 b) dargestellt sind, jeweils die ermittelte Abstrahlcharakteristik bzw. Ist-Abstrahlcharakteristik mit der zu erwartenden Soll-Abstrahlcharakteristik übereinstimmen, sofern tatsächlich eine Behandlung mit dem entsprechenden Fasertyp vorgesehen ist. Folglich würde in diesem Fall die Faser nach der Kalibration für eine weitere Verwendung freigegeben, da offenbar die korrekte Art von lichtleitender Faser 112 an der Lichtquelle 104 angeschlossen ist.

In der Figur 2 c) ist eine Kombination einer Positioniervorrichtung 110 für eine radial abstrahlende Faser 112 mit einer lichtleitenden Faser 112, welche zur frontalen Abstrahlung von elektromagnetischer Strahlung ausgebildet ist, dargestellt. Würde in dieser Kombination die lichtleitende Faser 112 mit elektromagnetischer Strahlung einer definierten Lichtleistung beaufschlagt, würde sowohl von der frontal angeordneten Photodiode 114 des Kalibrationsports 108 als auch von den seitlich angeordneten Photodioden 114 des Kalibrationsports 108 jeweils nur ein sehr geringer Photostrom detektiert. In frontaler Richtung würde die elektromagnetische Strahlung, welche aus der lichtleitenden Faser 112 ausgekoppelt wird, von der Positioniervorrichtung 110 gedämpft, während in radialer Richtung ein Austritt von elektromagnetischer Strahlung aus der lichtleitenden Faser aufgrund des durchgehenden Fasermantels 120 verhindert wird. Demzufolge würde die ermittelte Ist-Abstrahlcharakteristik nicht mit einer zu erwartenden Soll-Abstrahlcharakteristik für eine frontal oder radial abstrahlende Faser übereinstimmen. Folglich würde erfindungsgemäß die lichtleitende Faser 112 im Zuge der Kalibration nicht für weitere Verwendungen freigegeben werden, sondern vielmehr eine Fehlermeldung ausgegeben werden, welche darauf hinweist, dass entweder die falsche lichtleitende Faser 112 oder die falsche Positioniervorrichtung 110 verwendet wurde.

In der Figur 2 d) ist der zur Figur 2 c) orthogonale Fall dargestellt, bei dem eine radial abstrahlende lichtleitende Faser 112 in Kombination mit einer Positioniervorrichtung 110 verwendet wurde, die für die frontale Abstrahlung von elektromagnetischer Strahlung aus der lichtleitenden Faser 112 vorgesehen ist. Auch in diesem Fall würde bei Beaufschlagung der lichtleitenden Faser 112 mit elektromagnetischer Strahlung durch alle Photodioden 114 des Kalibrationsports 108 jeweils nur ein sehr geringer Photostrom detektiert, da in radialer Richtung die emittierte Strahlung durch die Positioniervorrichtung 110 gedämmt wird, während in frontaler Richtung die Ummantelung 120 der lichtleitenden Faser 112 eine Emission von elektromagnetischer Strahlung verhindern würde. Folglich würde auch hier bei einem Vergleich der ermittelten Ist-Abstrahlcharakteristik mit einer Soll-Abstrahlcharakteristik eine Diskrepanz zwischen den Charakteristika ermittelt werden, sodass die zur Kalibration vorgesehene Faser 112 nicht für eine weitere Verwendung freigegeben wird, sondern eine Fehlermeldung ausgegeben wird.

Würde beispielsweise durch eine gewählte Behandlungsmethode oder ein Behandlungsszenario vorgegeben, dass eine radial abstrahlende lichtleitende Faser 112 zu verwenden ist, jedoch die Abstrahlcharakteristik entsprechend der Figur 2 a) ermittelt werden, würde erfindungsgemäß die lichtleitende Faser 112 ebenfalls nicht für eine weitere Verwendung freigegeben werden. Analog hierzu würde für eine Anwendung, bei der eine frontal abstrahlende lichtleitende Faser 112 benötigt wird, bei Detektion der Abstrahlcharakteristik entsprechend Figur 2 b), ebenfalls keine Freigabe der lichtleitenden Faser 112 für eine weitere Verwendung erfolgen.

Wie zuvor bereits erwähnt wurde, kann sich je nach Anwendungsszenario die Länge des abstrahlenden Bereiches einer radial abstrahlenden lichtleitenden Faser 112 unterscheiden. Dabei ist die Positioniervorrichtung 110 vorzugsweise so ausgebildet, dass eine Positioniervorrichtung für unterschiedliche Längen des radial abstrahlenden Bereichs der lichtleitenden Faser 112 verwendet werden kann. Eine Unterscheidung von lichtleitenden Fasern 112 mit unterschiedlich langen radial abstrahlenden Bereichen ist dann unter Berücksichtigung der in die Faser 112 eingekoppelten Lichtleistung möglich.

Ist beispielsweise vorgesehen, dass in eine lichtleitende Faser 112, welche über eine Länge von 2 cm elektromagnetische Strahlung in radialer Richtung auskoppelt eine Eingangsleistung von 2 Watt eingekoppelt wird, ergibt sich hieraus eine bestimmte Abstrahlcharakteristik bezüglich der Verhältnisse der in verschiedene Raumrichtungen aus der Faser ausgekoppelten Lichtleistungen, welche von den Photodioden 114 des Kalibrationsports 108 gemessen werden. Würde jedoch irrtümlicherweise bei einer Eingangsleistung von 2 Watt eine Faser mit der Lichtquelle 104 verbunden, welche beispielsweise über eine Länge von 4 cm elektromagnetische Strahlung in radialer Richtung auskoppelt, würde die aus der Faser austretende Intensität nicht den erwarteten Werten für die eigentlich vorgesehene Faser entsprechen. Folglich würde durch die medizinische Vorrichtung 102 eine Fehlermeldung ausgegeben.

Es ist jedoch möglich, dass für eine Faser mit einem kürzeren oder längeren radial abstrahlenden Bereich dieselben Verhältnisse der radial ausgekoppelten Lichtleistungen bzw. Intensitäten von den Photodioden 114 des Kalibrationsports 108 gemessen werden. Hierzu müsste jedoch bei einem kürzeren radial abstrahlenden Bereich eine geringere Eingangsleistung bzw. bei einem längeren radial abstrahlenden Bereich eine höhere Eingangsleistung in die lichtleitende Faser 112 eingekoppelt werden. Folglich unterscheiden sich zwar möglicherweise die Verhältnisse der aus der Faser ausgekoppelten Lichtleistungen für verschiedene Fasertypen nicht, jedoch ist unter Berücksichtigung der in die Faser eingekoppelten Lichtleistung einer Unterscheidung der Fasertypen weiterhin möglich.

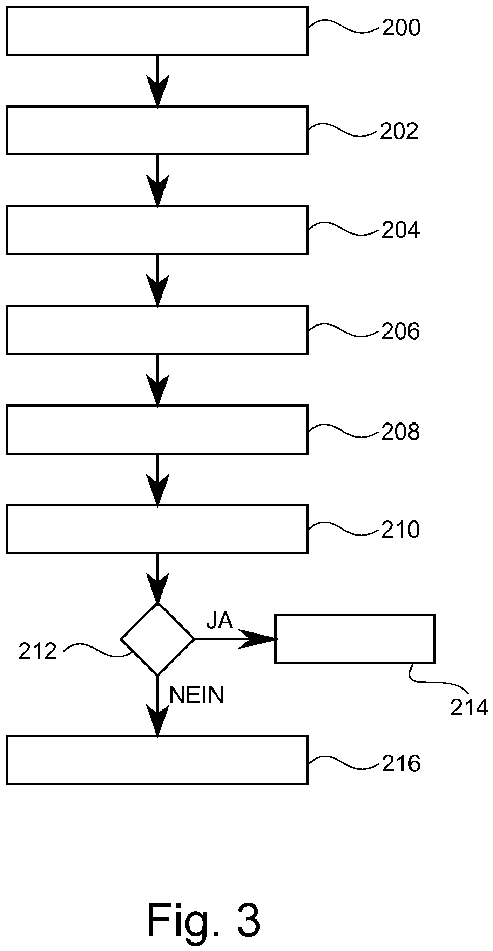

In Figur 3 ist ein Flussdiagramm des erfindungsgemäßen Verfahrens zur Kalibration einer Lichtquelle einer medizinischen Vorrichtung dargestellt. Hierzu kann beispielsweise das System 100, wie es in der Figur 1 dargestellt ist, verwendet werden. In einem ersten Verfahrensschritt 200 wird zunächst die lichtleitende Faser 112 mit der zu kalibrierenden Lichtquelle 104 der medizinischen Vorrichtung 102 verbunden. Ferner wird eine Positioniervorrichtung 110, wie sie beispielsweise in Figur 2 dargestellt ist, in Schritt 202 in den Kalibrationsport 108 der medizinischen Vorrichtung 102 eingesetzt. Dabei ist anzumerken, dass der Kalibrationsport 108 nicht zwingend in der medizinischen Vorrichtung 102 ausgebildet sein muss. Es kann sich bei dem Kalibrationsport 108 vielmehr auch um ein separates Element handeln, welches über eine entsprechende Datenverbindung mit der medizinischen Vorrichtung 102 so verbunden ist, dass von dem Kalibrationsport 108 ermittelte Ist-Abstrahlcharakteristika an die medizinische Vorrichtung 102 übermittelt werden können.

Nachdem die lichtleitende Faser 112 mit der Lichtquelle 104 verbunden wurde und die Positioniervorrichtung 110 in dem Kalibrationsport 108 eingesetzt wurde, wird im Verfahrensschritt 204 die lichtleitende Faser in den Aufnahmekanal der Positioniervorrichtung eingeführt. Hierzu weist die Positioniervorrichtung 110 vorzugsweise einen trichterförmigen Verlauf an einer ersten aus dem Kalibrationsport 108 hervorstehenden Stirnseite auf, sodass die lichtleitende Faser 112 leicht in den für die Faser vorgesehenen Kanal der Positioniervorrichtung eingeführt werden kann. Dabei muss die Faser 112 bis zum Anschlag in die Positioniervorrichtung 110 eingeführt werden, sodass die jeweils abstrahlenden Bereiche der lichtleitenden Faser 112 in den entsprechenden Bereichen bzw. im Bereich der Ausnehmungen 118 der Positioniervorrichtung 110 zu liegen kommen. Andernfalls würde möglicherweise trotz einer korrekt gewählten Kombination von lichtleitender Faser 112 und Positioniervorrichtung eine fehlerhafte Abstrahlcharakteristik ermittelt werden.

Beispielsweise ausgelöst durch eine entsprechende Betätigung der medizinischen Vorrichtung 102 über die Bedienelemente 106 wird dann in Schritt 206 elektromagnetischer Strahlung einer definierten Lichtleistung durch die Lichtquelle 104 erzeugt und in die lichtleitende Faser 112 über eine entsprechende Faserverbindung eingekoppelt. Die in die lichtleitende Faser 112 eingekoppelte Strahlung wird dabei im Bereich des Kalibrationsports aus den entsprechenden Bereichen der lichtleitenden Faser 112 ausgekoppelt, sodass im Verfahrensschritt 208 die räumliche Abstrahlcharakteristik der aus der lichtleitenden Faser 112 im Bereich des Kalibrationsports 108 ausgekoppelten Lichtleistung als Ist-Abstrahlcharakteristik mittels der Photodioden 114 des Kalibrationsports 108 ermittelt werden kann.

Anschließend wird in Schritt 210 eine Soll-Abstrahlcharakteristik für die lichtleitende Faser 112 ermittelt. Dabei kann beispielsweise vorgesehen sein, dass von der medizinischen Vorrichtung 102 aufgrund der Angabe einer gewünschten Behandlungsart vorgegeben wird, welcher Typ von lichtleitender Faser 112 mit der Lichtquelle 104 zu verbinden ist. Dabei wird die Soll-Abstrahlcharakteristik aufgrund der Information bezüglich des zu verwendeten Fasertyps ausgewählt. Alternativ kann auch die lichtleitende Faser 112 selbst mit einem Identifikationsmerkmal, beispielsweise einem RFID-Chip ausgestattet sein, welcher durch ein entsprechendes Lesegerät an der Verbindung zwischen Lichtquelle 104 und lichtleitende Faser 112 ausgelesen wird. In diesem Fall kann die medizinische Vorrichtung 102 selbstständig ermitteln, welche lichtleitende Faser 112 bzw. welcher Fasertyp an der Lichtquelle 104 angeschlossen wurde.

Die Soll-Abstrahlcharakteristik wird dann auf Grundlage des ermittelten Fasertyps bestimmt und kann beispielsweise aus einem Speichermedium, welches in der medizinischen Vorrichtung 102 vorgehalten wird, ausgelesen werden.

In dem nachfolgenden Verfahrensschritt 212 wird dann die ermittelte Ist-Abstrahlcharakteristik mit der ermittelten Soll-Abstrahlcharakteristik verglichen. Wird dabei ermittelt, dass die Ist-Abstrahlcharakteristik der Soll-Abstrahlcharakteristik entspricht, wird die lichtleitende Faser 112 für weitere Verwendungen freigeben. Dies erfolgt im Verfahrensschritt 214. Die Entsprechung der Ist-Abstrahlcharakteristik zur Soll-Abstrahlcharakteristik kann dabei unter Berücksichtigung einer Toleranz ermittelt werden, sodass die Soll-Abstrahlcharakteristik und die Ist-Abstrahlcharakteristik nicht identisch sein müssen, jedoch in gewissen Toleranzen übereinstimmen sollen. Bei der weiteren Verwendung der lichtleitenden Faser kann es sich beispielsweise um die direkte Behandlung eines Patienten handeln oder es kann zuvor die in die lichtleitende Faser eingekoppelte Lichtleistung so angepasst werden, dass die aus der lichtleitenden Faser ausgekoppelte Intensität der für eine Behandlung notwendigen Intensität entspricht. Wurde im Schritt 212 hingegen ermittelt, dass die Ist-Abstrahlcharakteristik der Soll-Abstrahlcharakteristik nicht entspricht, also außerhalb der eventuell vorhandenen Toleranzen liegt, wird im Schritt 216 eine Fehlermeldung ausgegeben, welche anzeigt, dass die Kalibration der Faser 112 nicht erfolgreich war. Dabei kann die Fehlermeldung beispielsweise auch anzeigen, dass ein falscher Fasertyp, oder eine falsche Positioniervorrichtung 110 verwendet wurde, oder dass für eine gegebene eingekoppelte Lichtleistung die aus der lichtleitenden Faser 112 austretende Lichtleistung deutlich zu niedrig ist, was auf einen Defekt der lichtleitenden Faser 112 oder der Fasereinkopplung hinweisen kann, oder deutlich zu hoch ist, was ebenfalls auf einen Defekt, oder eine falsch gewählte Länge des radial abstrahlenden Bereichs einer lichtleitenden Faser 112 hinweisen kann. Dabei kann die Fehlermeldung eine entsprechende Information enthalten.

Bei Verwendung mehrerer lichtleitender Fasern 112, welche jeweils mit einer Lichtquelle 104 der medizinischen Vorrichtung verbunden sind, kann das zuvor beschriebene Verfahren für jede der Fasern 112 einzeln wiederholt werden. Dabei kann nach einer Ausführungsform vorgesehen sein, dass eine bereits freigegebene Faser 112 nach der Kalibration weiterhin Licht im Bereich des für den Menschen sichtbaren Spektrums emittiert, sodass durch einen Benutzer leicht erkannt werden kann, ob eine Faser 112 bereits kalibriert wurde, oder nicht.

Die Erfindung ist nicht auf eine der vorbeschriebenen Ausführungsformen beschränkt, sondern in vielfältiger Weise abwandelbar.

So kann anstelle von drei Photodioden 114 eine im Wesentlichen beliebige Zahl von Photodioden 114 in einem Kalibrationsport 108 vorgesehen sein, wobei wenigstens eine Photodiode seitlich an dem Kalibrationsport angeordnet ist, sowie wenigstens eine Photodiode an einem longitudinalen Ende des Kalibrationsports angeordnet ist. Dabei kann durch eine höhere Zahl von Photodioden 114 eine bessere bzw. genauere Erfassung einer räumlichen Abstrahlcharakteristik einer lichtleitenden Faser 112 erfolgen.

Weiter können auch die Photodioden 114 durch andere Sensormittel, wie beispielsweise CCD- oder CMOS-Sensoren ersetzt sein. Durch Verwendung von derartigen Sensoren könnte beispielsweise ein detailliertes Bild der aus der Positioniervorrichtung austretenden elektromagnetischen Strahlung ermittelt werden, aus dem ebenfalls abgeleitet werden kann, welcher Fasertyp aktuell im Zuge eines Kalibrationsprozesses verwendet wird.

Zuvor wurde weiter ausgeführt, das im Wesentlichen zwei Arten von Positioniervorrichtungen 110 vorgesehen sind, nämlich solche, welche eine Abstrahlung elektromagnetischer Strahlung in radialer Richtung erlauben, oder solche, die eine Abstrahlung elektromagnetischer Strahlung in longitudinaler Richtung erlauben. Es ist jedoch im Rahmen der vorliegenden Erfindung durchaus möglich, eine Positioniervorrichtung 110 zu verwenden, welche für beide Abstrahlungsrichtungen gleichzeitig geeignet ist. Die Positioniervorrichtung 110 muss lediglich so gestaltet sein, dass eine räumliche Abstrahlcharakteristik einer in der Positioniervorrichtung angeordneten lichtleitenden Faser 112 ermittelt werden kann.

### Bezugszeichenliste

- 100: System
- 102: Medizinische Vorrichtung
- 104: Lichtquelle
- 106: Bedienelement
- 108: Kalibrationsport
- 110: Positioniervorrichtung
- 112: lichtleitende Faser
- 114: Photodiode
- 116: Faserkern
- 118: Ausnehmung
- 120: Fasermantel

## Patentansprüche

1. Verfahren zur Kalibration einer Lichtquelle (104) einer medizinischen Vorrichtung (102), wobei die Lichtquelle (104) mit wenigstens einer lichtleitenden Faser (112) so verbindbar ist, dass von der Lichtquelle (104) erzeugte elektromagnetische Strahlung einer definierten Lichtleistung in die lichtleitende Faser (112) zumindest teilweise eingekoppelt wird, wobei die medizinische Vorrichtung (102) mit wenigstens einem Kalibrationsport (108) verbunden ist, wobei der Kalibrationsport (108) Sensormittel zur Ermittlung einer räumlichen Abstrahlcharakteristik einer in den Kalibrationsport (108) eingeführten lichtleitenden Faser (112) aufweist, wobei das Verfahren die nachfolgenden Schritte aufweist:
a) Verbinden der lichtleitenden Faser (112) mit der Lichtquelle (104),
b) Einsetzen einer Positioniervorrichtung (110) für die lichtleitende Faser (112) in den Kalibrationsport (108), wobei die Positioniervorrichtung (110) einen Aufnahmekanal zur Aufnahme der lichtleitenden Faser (112) sowie wenigstens eine Abstrahlöffnung aufweist, wobei die wenigstens eine Abstrahlöffnung die Transmission einer aus der lichtleitenden Faser (112) ausgekoppelten Lichtleistung in wenigstens einer definierten Raumrichtung ermöglicht,
c) Einführen der lichtleitenden Faser (112) in den Aufnahmekanal der Positioniervorrichtung (110),
d) Einkoppeln von elektromagnetischer Strahlung einer definierten Lichtleistung in die lichtleitende Faser (112),
e) Ermitteln der räumlichen Abstrahlcharakteristik der aus der lichtleitenden Faser (112) im Bereich des Kalibrationsports (108) ausgekoppelten Lichtleistung als Ist-Abstrahlcharakteristik,
f) Ermitteln einer Soll-Abstrahlcharakteristik der aus der lichtleitenden Faser (112) im Bereich des Kalibrationsports (108) ausgekoppelten Lichtleistung für die in die lichtleitende Faser (112) eingekoppelte Lichtleistung mittels der Sensormittel,
g) Vergleichen der ermittelten Ist-Abstrahlcharakteristik mit der ermittelten Soll-Abstrahlcharakteristik,
h) Wenn die Ist-Abstrahlcharakteristik der Soll-Abstrahlcharakteristik entspricht, Freigeben der lichtleitenden Faser (112) zur weiteren Verwendung, und
i) Wenn die Ist-Abstrahlcharakteristik der Soll-Abstrahlcharakteristik nicht entspricht, Ausgeben einer Fehlermeldung
**dadurch gekennzeichnet, dass**
die Sensormittel wenigstens eine seitliche Photodiode (114) aufweisen, die seitlich an dem Kalibrationsport (108) angeordnet ist, sowie wenigstens eine frontale Photodiode (114), die an einem longitudinalen Ende des Kalibrationsports (108) angeordnet ist, wobei das Ermitteln der Ist-Abstrahlcharakteristik das Ermitteln des in den Photodioden (114) durch die von der lichtleitenden Faser (112) ausgekoppelte Lichtleistung erzeugten, jeweiligen Photostroms umfasst, und
wobei das Vergleichen der ermittelten Ist-Abstrahlcharakteristik mit der ermittelten Soll-Abstrahlcharakteristik das Vergleichen der ermittelten Photoströme mit in der Soll-Abstrahlcharakteristik enthaltenen Photoströmen für die jeweiligen Photodioden (114) umfasst.

2. Verfahren nach Anspruch 1, **dadurch gekennzeichnet, dass** es sich bei einer lichtleitenden Faser (112) um eine Faser (112) eines ersten Fasertyps oder eines zweiten Fasertyps handelt, wobei der erste Fasertyp dazu ausgebildet ist, in die lichtleitende Faser (112) eingekoppelte elektromagnetische Strahlung in Längsrichtung der Faser (112) zu emittieren und wobei der zweite Fasertyp dazu ausgebildet ist, in die lichtleitende Faser (112) eingekoppelte elektromagnetische Strahlung quer zur Längsrichtung der Faser (112) über eine definierte Länge der Faser (112) zu emittieren, wobei das Verfahren ferner das Ermitteln des verwendeten Fasertyps aufweist, wobei die Ermittlung der Soll-Abstrahlcharakteristik den ermittelten Fasertyp berücksichtigt.

3. Verfahren nach Anspruch 2, **dadurch gekennzeichnet, dass** das Ermitteln des verwendeten Fasertyps eine Nutzereingabe umfasst.

4. Verfahren nach Anspruch 2 oder 3, **dadurch gekennzeichnet, dass** das Verfahren ferner das Ermitteln der in die lichtleitende Faser (112) einzukoppelnden, definierten Lichtleistung auf Grundlage des an die Lichtquelle (104) angeschlossenen Fasertyps beinhaltet.

5. Verfahren nach Anspruch 1, **dadurch gekennzeichnet, dass** wenn die Verhältnisse der Photoströme der Ist-Abstrahlcharakteristik den Verhältnissen der Photoströme der Soll-Abstrahlcharakteristik entsprechen, die Photoströme der Ist-Abstrahlcharakteristik jedoch geringer sind als die entsprechenden Photoströme der Soll-Charakteristik, die Fehlermeldung einen Defekt der lichtleitenden Faser (112) und/oder der Kopplung zwischen der Lichtleitenden Faser (112) und der Lichtquelle (104) und/oder eine fehlerhafte abstrahlende Fläche der lichtleitenden Faser (112) anzeigt.

6. Verfahren nach einem der vorherigen Ansprüche, **dadurch gekennzeichnet, dass** die medizinische Vorrichtung (102) wenigstens zwei Lichtquellen (104) aufweist, die jeweils mit einer lichtleitenden Faser (112) so verbindbar sind, dass von den Lichtquellen (104) erzeugte elektromagnetische Strahlung jeweils in die mit einer Lichtquelle (104) verbundene lichtleitende Faser (112) zumindest teilweise eingekoppelt wird, wobei wenigstens die Verfahrensschritte a) und c) bis i) individuell für die mit den Lichtquellen (104) verbundenen lichtleitenden Fasern (112) durchgeführt werden.

7. Verfahren nach einem der vorherigen Ansprüche, **dadurch gekennzeichnet, dass** nach der Freigabe einer Faser (112), die mit der Faser (112) verbundene Lichtquelle (104) für eine definierte Zeitspanne Licht einer Wellenlänge zwischen 350nm und 850nm in die Faser (112) einkoppelt.

8. Medizinische Vorrichtung (102) mit wenigstens einer Lichtquelle (104), wobei die Lichtquelle (104) mit wenigstens einer lichtleitenden Faser (112) so verbindbar ist, dass von der Lichtquelle (104) erzeugte elektromagnetische Strahlung einer definierten Lichtleistung in die lichtleitende Faser (112) zumindest teilweise eingekoppelt wird, wobei die medizinische Vorrichtung (102) mit wenigstens einem Kalibrationsport (108) verbunden ist, wobei der Kalibrationsport (108) Sensormittel zu Ermittlung einer räumlichen Abstrahlcharakteristik einer in den Kalibrationsport (108) eingeführten lichtleitenden Faser (112) aufweist, wobei die Vorrichtung (102) dazu ausgebildet ist:
• elektromagnetische Strahlung einer definierten Lichtleistung in eine mit der Vorrichtung (102) verbundene und in dem Kalibrationsport (108) positionierte lichtleitende Faser (112) einzukoppeln,
• Die räumliche Abstrahlcharakteristik der aus der lichtleitenden Faser (112) im Bereich des Kalibrationsports (108) ausgekoppelten Lichtleistung als Ist-Abstrahlcharakteristik zu ermitteln,
• Eine Soll-Abstrahlcharakteristik der aus der lichtleitenden Faser (112) im Bereich des Kalibrationsports (108) ausgekoppelten Lichtleistung für die in die lichtleitende Faser (112) eingekoppelte Lichtleistung zu ermitteln,
• Die ermittelte Ist-Abstrahlcharakteristik mit der ermittelten Soll-Abstrahlcharakteristik zu vergleichen,
• Wenn die Ist-Abstrahlcharakteristik der Soll-Abstrahlcharakteristik entspricht, die lichtleitende Faser (112) zur weiteren Verwendung freizugeben, und
• Wenn die Ist-Abstrahlcharakteristik der Soll-Abstrahlcharakteristik nicht entspricht, eine Fehlermeldung auszugeben
**dadurch gekennzeichnet, dass**
die Sensormittel wenigstens eine seitliche Photodiode (114) aufweisen, die seitlich an dem Kalibrationsport (108) angeordnet ist, sowie wenigstens eine frontale Photodiode (114), die an einem longitudinalen Ende des Kalibrationsports (108) angeordnet ist, wobei die Vorrichtung (102) zum Ermitteln der Ist-Abstrahlcharakteristik dazu ausgebildet ist, den in den Photodioden (114) durch die von der lichtleitenden Faser (112) ausgekoppelte Lichtleistung erzeugten, jeweiligen Photostrom zu ermitteln, undwobei die Vorrichtung (102) zum Vergleichen der ermittelten Ist-Abstrahlcharakteristik mit der ermittelten Soll-Abstrahlcharakteristik dazu ausgebildet ist, die ermittelten Photoströme mit in der Soll-Abstrahlcharakteristik enthaltenen Photoströmen für die jeweiligen Photodioden (114) zu vergleichen.

## Claims

1. Method for calibrating a light source (104) of a medical device (102), wherein the light source (104) can be connected to at least one light-conducting fibre (112) in such a way that electromagnetic radiation of a defined light output, which is generated by the light source (104), is at least partially coupled into the light-conducting fibre (112), wherein the medical device (102) is connected to at least one calibration port (108), the calibration port (108) including sensor means for determining a spatial radiation pattern of a light-conducting fibre (112) introduced into the calibration port (108), the method comprising the following steps:
a) connecting the light-conducting fibre (112) to the light source (104),
b) inserting a positioning device (110) for the light-conducting fibre (112) into the calibration port (108), the positioning device (110) having a receiving channel for receiving the light-conducting fibre (112) and at least one radiation aperture, the at least one radiation aperture enabling the transmission of a light output coupled out of the light-conducting fibre (112) in at least one defined spatial direction,
c) inserting the light-conducting fibre (112) into the receiving channel of the positioning device (110),
d) coupling electromagnetic radiation of a defined light output into the light-conducting fibre (112),
e) determining the spatial radiation pattern of the light output coupled out of the light-conducting fibre (112) in the region of the calibration port (108) as an actual radiation pattern,
f) determining a desired radiation pattern of the light output coupled out of the light-conducting fibre (112) in the region of the calibration port (108) for the light output coupled into the light-conducting fibre (112) by means of the sensor means,
g) comparing the determined actual radiation pattern with the determined desired radiation pattern,
h) releasing, if the actual radiation pattern corresponds to the desired radiation pattern, the light-conducting fibre (112) for further use, and
i) outputting an error message if the actual radiation pattern does not correspond to the desired radiation pattern,
**characterized in that**
the sensor means comprise at least one lateral photodiode (114) located laterally on the calibration port (108) and at least one frontal photodiode (114) located at a longitudinal end of the calibration port (108), wherein determining the actual radiation pattern comprises determining the respective photocurrent generated in the photodiodes (114) by the light output coupled out from said light-conducting fibre (112), and
wherein comparing the determined actual radiation pattern with the determined desired radiation pattern comprises comparing the determined photocurrents with photocurrents included in the desired radiation pattern for the respective photodiodes (114).

2. Method according to claim 1, **characterized in that** a light-conducting fibre (112) is a fibre (112) of a first fibre type or of a second fibre type, the first fibre type being adapted to emit electromagnetic radiation coupled into the light-conducting fibre (112) in the longitudinal direction of the fibre (112), and the second fibre type being adapted to emit electromagnetic radiation coupled into the light-conducting fibre (112) transversely to the longitudinal direction of the fibre (112) over a defined length of the fibre (112), the method further comprising determining the type of fibre used, the determination of the desired radiation pattern taking into account the determined fibre type.

3. Method according to claim 2, **characterized in that** determining the type of fibre used comprises a user input.

4. Method according to claim 2 or 3, **characterized in that** the method further includes determining the defined light output to be coupled into the light-conducting fibre (112) based on the type of fibre connected to the light source (104).

5. Method according to claim 1, **characterized in that** when the ratios of the photocurrents of the actual radiation pattern correspond to the ratios of the photocurrents of the desired radiation pattern, but the photocurrents of the actual radiation pattern are less than the corresponding photocurrents of the desired pattern, the error message indicates a defect of the light-conducting fibre (112) and/or the coupling between the light-conducting fibre (112) and the light source (104) and/or a defective radiating surface of the light-conducting fibre (112).

6. Method according to any one of the preceding claims, **characterized in that** the medical device (102) comprises at least two light sources (104), each of which can be connected to a light-conducting fibre (112) in such a way that electromagnetic radiation generated by the light sources (104) is in each case at least partially coupled into the light-conducting fibre (112) connected to a light source (104), wherein at least the method steps a) and c) to i) are carried out individually for the light-conducting fibres (112) connected to the light sources (104).

7. Method according to any one of the preceding claims, **characterized in that** after the release of a fibre (112), the light source (104) connected to the fibre (112) couples light of a wavelength between 350nm and 850nm into the fibre (112) for a defined period of time.

8. Medical device (102) comprising at least one light source (104), wherein the light source (104) can be connected to at least one light-conducting fibre (112) in such a way that electromagnetic radiation of a defined light output, which is generated by the light source (104), is at least partially coupled into the light-conducting fibre (112), wherein the medical device (102) is connected to at least one calibration port (108), the calibration port (108) having sensor means for determining a spatial radiation pattern of a light-conducting fibre (112) introduced into the calibration port (108), the device (102) being designed for:
- coupling electromagnetic radiation of a defined light output into a light-conducting fibre (112) connected to the device (102) and positioned in the calibration port (108),
- determining the spatial radiation pattern of the light output coupled out of the light-conducting fibre (112) in the region of the calibration port (108) as an actual radiation pattern,
- determining a desired radiation pattern of the light output coupled out of the light-conducting fibre (112) in the region of the calibration port (108) for the light output coupled into the light-conducting fibre (112),
- comparing the determined actual radiation pattern with the determined desired radiation pattern,
- releasing, if the actual radiation pattern corresponds to the desired radiation pattern, the light-conducting fibre (112) for further use, and
- outputting an error message if the actual radiation pattern does not correspond to the desired radiation pattern,
**characterized in that**
the sensor means comprises at least one lateral photodiode (114) located laterally on the calibration port (108) and at least one frontal photodiode (114) located at a longitudinal end of the calibration port (108), wherein the device (102) for determining the actual radiation pattern is adapted to the respective photocurrent generated in the photodiodes (114) by the light output from the light-conducting fibre (112), and wherein the device (102) for comparing the determined actual radiation pattern with the determined desired radiation pattern is adapted to compare the determined photocurrents with photocurrents included in the desired radiation pattern for the respective photodiodes (114).

## Revendications

1. Procédé servant à étalonner une source de lumière (104) d'un dispositif médical (102), dans lequel la source de lumière (104) peut être reliée à au moins une fibre de guidage de lumière (112) de sorte qu'un rayonnement électromagnétique généré par la source de lumière (104) d'une puissance de lumière définie est injecté au moins partiellement dans la fibre de guidage de lumière (112), dans lequel le dispositif médical (102) est relié à au moins un port d'étalonnage (108), dans lequel le port d'étalonnage (108) présente des moyens de capteur servant à déterminer une caractéristique spatiale d'émission de rayonnement d'une fibre de guidage de lumière (112) introduite dans le port d'étalonnage (108), dans lequel le procédé comprend les étapes suivantes :
a) une liaison de la fibre de guidage de lumière (112) à la source de lumière (104) ;
b) une insertion d'un dispositif de positionnement (110) pour la fibre de guidage de lumière (112) dans le port d'étalonnage (108), dans lequel le dispositif de positionnement (110) présente un canal de réception servant à recevoir la fibre de guidage de lumière (112) et au moins un orifice d'émission de rayonnement, dans lequel le au moins un orifice d'émission de rayonnement permet une transmission d'une puissance de lumière sortie de la fibre de guidage de lumière (112) dans au moins une direction spatiale définie,
c) une introduction de la fibre de guidage de lumière (112) dans le canal de réception du dispositif de positionnement (110),
d) une injection d'un rayonnement électromagnétique d'une puissance de lumière définie dans la fibre de guidage de lumière (112),
e) une détermination de la caractéristique spatiale d'émission de rayonnement de la puissance de lumière sortie de la fibre de guidage de lumière (112) dans la zone du port d'étalonnage (108) en tant que caractéristique d'émission de rayonnement réelle ;
f) une détermination d'une caractéristique d'émission de rayonnement théorique de la puissance de lumière sortie de la fibre de guidage de lumière (112) dans la zone du port d'étalonnage (108) pour la puissance de lumière injectée dans la fibre de guidage de lumière (112) au moyen des moyens de capteur ;
g) une comparaison de la caractéristique d'émission de rayonnement réelle déterminée à la caractéristique d'émission de rayonnement théorique déterminée,
h) si la caractéristique d'émission de rayonnement réelle correspond à la caractéristique d'émission de rayonnement théorique, un déblocage de la fibre de guidage de lumière (112) pour poursuivre l'utilisation ; et
i) si la caractéristique d'émission de rayonnement réelle ne correspond pas à la caractéristique d'émission de rayonnement théorique, un envoi d'un message d'erreur
**caractérisé en ce que**,
les moyens de capteur présentent au moins une photodiode latérale (114) qui est agencée latéralement sur le port d'étalonnage (108), et au moins une photodiode frontale (114) qui est agencée à une extrémité longitudinale du port d'étalonnage (108),
dans lequel la détermination de la caractéristique d'émission de rayonnement réelle comprend une détermination du courant photoélectrique respectif généré dans les photodiodes (114) par la puissance de lumière sortie de la fibre de guidage de lumière (112), et
dans lequel la comparaison de la caractéristique d'émission de rayonnement réelle déterminée avec la caractéristique d'émission de rayonnement théorique déterminée présente une comparaison des courants photoélectriques déterminés avec des courants photoélectriques contenus dans la caractéristique d'émission de rayonnement théoriques pour les photodiodes respectives (114).

2. Procédé selon la revendication 1, **caractérisé en ce qu'**une fibre de guidage de lumière (112) est une fibre (112) d'un premier type de fibre ou d'un second type de fibre, dans lequel le premier type de fibre est réalisé pour émettre un rayonnement électromagnétique injecté dans la fibre de guidage de lumière (112) dans la direction longitudinale de la fibre (112), et dans lequel le second type de fibre est réalisé pour émettre un rayonnement électromagnétique injecté dans la fibre de guidage de lumière (112) transversalement par rapport à la direction longitudinale de la fibre (112) sur une longueur définie de la fibre (112), dans lequel le procédé présente en outre une détermination du type de fibre utilisé, dans lequel la détermination de la caractéristique d'émission de rayonnement théorique tient compte du type de fibre déterminé.

3. Procédé selon la revendication 2, **caractérisé en ce que** la détermination du type de fibre utilisée comprend une instruction d'utilisateur.

4. Procédé selon la revendication 2 ou 3, **caractérisé en ce que** le procédé contient en outre une détermination de la puissance de lumière définie à injecter dans la fibre de guidage de lumière (112) sur la base du type de fibre relié à la source de lumière (104).

5. Procédé selon la revendication 1, **caractérisé en ce que** lorsque les rapports des courants photoélectriques de la caractéristique d'émission de rayonnement réelle correspondent aux rapports des courants photoélectriques de la caractéristique d'émission de rayonnement théorique, les courants photoélectriques de la caractéristique d'émission de rayonnement réelle sont inférieurs aux courants photoélectriques correspondants de la caractéristique d'émission de rayonnement théorique, le message d'erreur indique un défaut dans la fibre de guidage de lumière (112) et/ou le couplage entre la fibre de guidage de lumière (112) et la source de lumière (104) et/ou une surface d'émission de rayonnement défectueuse de la fibre de guidage de lumière (112).

6. Procédé selon l'une quelconque des revendications précédentes, **caractérisé en ce que** le dispositif médical (102) présente au moins deux sources de lumière (104) qui peuvent être reliées chacune à une fibre de guidage de lumière (112), **en ce que** le rayonnement électromagnétique généré par la source de lumière (104) est respectivement au moins partiellement injecté dans la fibre de guidage de lumière (112) reliée à une source de lumière (104), dans lequel au moins les étapes de procédé a) et c) à i) sont effectuées individuellement pour les fibres de guidage de lumière (112) reliées aux sources de lumière (104).

7. Procédé selon l'une quelconque des revendications précédentes, **caractérisé en ce que** la source de lumière (104) reliée à la fibre (112), après la libération d'une fibre (112), injecte de la lumière d'une longueur d'onde comprise entre 350 nm et 850 nm dans la fibre (112) pendant une durée définie.

8. Dispositif médical (102) avec au moins une source de lumière (104), dans lequel la source de lumière (104) peut être reliée à au moins une fibre de guidage de lumière (112) de sorte qu'un rayonnement électromagnétique généré par la source de lumière (104) d'une puissance de lumière définie est injecté au moins partiellement dans la fibre de guidage de lumière (112), dans lequel le dispositif médical (102) est relié à au moins un port d'étalonnage (108), dans lequel le port d'étalonnage (108) présente des moyens de capteur servant à déterminer une caractéristique spatiale d'émission de rayonnement d'une fibre de guidage de lumière (112) introduite dans le port d'étalonnage (108), dans lequel le dispositif (102) est réalisé pour :
• une injection d'un rayonnement électromagnétique d'une puissance de lumière définie dans une fibre de guidage de lumière (112) reliée au dispositif (102) et positionnée dans le port d'étalonnage (108),
• une détermination de la caractéristique spatiale d'émission de la puissance de lumière sortie de la fibre de guidage de lumière (112) dans la zone du port d'étalonnage (108) en tant que caractéristique d'émission de rayonnement réelle,
• une détermination d'une caractéristique d'émission de rayonnement théorique de la puissance de lumière sortie de la fibre de guidage de lumière (112) dans la zone du port d'étalonnage (108) pour la puissance de lumière injectée dans la fibre de guidage de lumière (112),
• une comparaison de la caractéristique d'émission de rayonnement réelle déterminée à la caractéristique d'émission de rayonnement théorique déterminée,
• si la caractéristique d'émission de rayonnement réelle correspond à la caractéristique d'émission de rayonnement théorique, une libération de la fibre de guidage de lumière (112) pour une utilisation ultérieure, et
• si la caractéristique d'émission de rayonnement réelle ne correspond pas à la caractéristique d'émission de rayonnement théorique, un envoi d'un message d'erreur
**caractérisé en ce que**,
les moyens de capteur présentent au moins une photodiode latérale (114) qui est agencée latéralement sur le port d'étalonnage (108), et au moins une photodiode frontale (114) qui est agencée à une extrémité longitudinale du port d'étalonnage (108),
dans lequel le dispositif est réalisé pour déterminer la caractéristique d'émission de rayonnement réelle du courant photoélectrique respectif généré dans les photodiodes (114) par la puissance de lumière sortie de la fibre de guidage de lumière (112), et
dans lequel le dispositif (102) est réalisé pour comparer la caractéristique d'émission de rayonnement réelle déterminée avec la caractéristique d'émission de rayonnement théorique déterminée, pour comparer les courants photoélectriques déterminés avec des courants photoélectriques contenus dans la caractéristique d'émission de rayonnement théorique pour les photodiodes respectives (114).
